# EUROPEAN PATENT SPECIFICATION

(11) **EP 4 076 664 B1**
(45) Date of publication and mention of the grant of the patent: **30.08.2023**
(21) Application number: 20833855.8
(22) Date of filing: 18.12.2020
(51) Int. Cl.: A61P 35/00, C07D 487/04, A61K 31/454

(54) **EGFR INHIBITORS**
EGFR-INHIBITOREN
INHIBITEURS D' EGFR

(30) Priority: 20.12.2019 EP 19218403
(43) Date of publication of application: 26.10.2022
(73) Proprietor: F. Hoffmann-La Roche AG, 4070 Basel (CH)
(72) Inventor: JAESCHKE, Georg, 4070 Basel (CH); KUHN, Bernd, 4070 Basel (CH); NAGEL, Yvonne Alice, 4070 Basel (CH); RICCI, Antonio, 4070 Basel (CH)
(74) Representative: Salud, Carlos E.
(86) International application number: PCT/EP2020/086914
(87) International publication number: WO 2021/123084

(56) References cited:
- WO-A1-2017/004383
- WO-A1-2018/115218
- WO-A1-2018/220149
- WO-A1-2019/149922
- YONG JIA ET AL: "Overcoming EGFR(T790M) and EGFR(C797S) resistance with mutant-selective allosteric inhibitors", NATURE, vol. 534, no. 7605, 25 May 2016 (2016-05-25), pages 129-132, XP055342543, London ISSN: 0028-0836, DOI: 10.1038/nature17960

## Description

The present invention provides a compound that is selective allosteric inhibitors of T790M/L858R, T790M/L858R/C797S, L858R, L858R/C797S containing EGFR mutants, its manufacture and pharmaceutical compositions containing it. Disclosed but not claimed is its use as a therapeutically active substance.

The present invention provides a novel compound of formula (I) 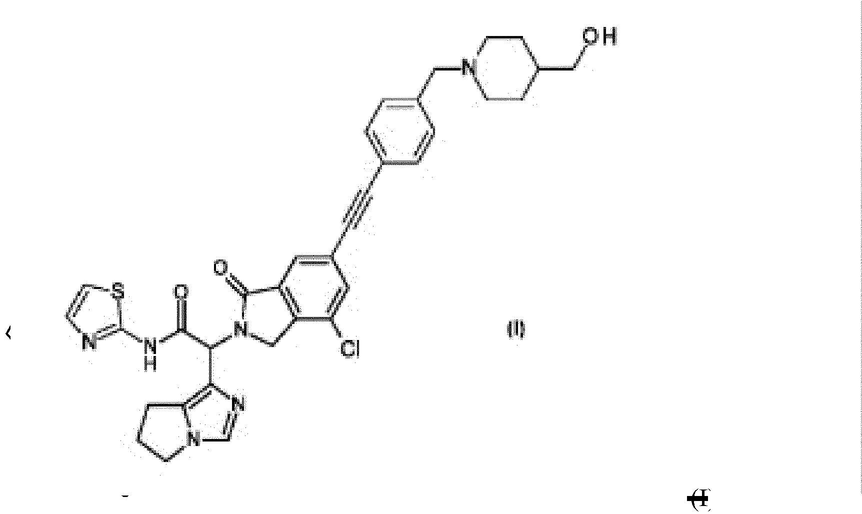 or a pharmaceutically acceptable salt thereof.

The HER family receptor tyrosine kinases are mediators of cell growth, differentiation and survival. The receptor family includes four distinct members, i.e. epidermal growth factor receptor (EGFR, ErbBl, or HER1) HER2 (ErbB2), HER3 (ErbB3) and HER4 (ErbB4). Upon ligand binding the receptors form homo and heterodimers and subsequent activation of the intrinsic tyrosine kinase activity leads to receptor auto-phosphorylation and the activation of downstream signaling molecules (Yarden, Y., Sliwkowski, MX. Untangling the ErbB signalling network. Nature Review Mol Cell Biol. 2001 Feb;2(2): 127-37). De-regulation of EGFR by overexpression or mutation has been implicated in many types of human cancer including colorectal, pancreatic, gliomas, head and neck and lung cancer, in particular non-small cell lung cancer (NSCLC) and several EGFR targeting agents have been developed over the years (Ciardiello, F., and Tortora, G. (2008). EGFR antagonists in cancer treatment. The New England journal of medicine 358, 1160-1174). Erlotinib (Tarceva^{®}), a reversible inhibitor of the EGFR tyrosine kinase was approved in numerous countries for the treatment of recurrent NSCLC.

An impressive single agent activity of EGFR tyrosine kinase inhibitors is observed in a subset of NSCLC patients whose tumors harbor somatic kinase domain mutations, whereas clinical benefit in wild-type EGFR patients is greatly diminished (Paez, J. et al. (2004). EGFR mutations in lung cancer: correlation with clinical response to gefitinib therapy. Science (New York, NY 304, 1497-1500). The most common somatic mutations of EGFR are exon 19 deletions with delta 746-750 the most prevalent mutation and the exon 21 amino acid substitutions with L858R the most frequent mutation (Sharma SV, Bell DW, Settleman J, Haber DA. Epidermal growth factor receptor mutations in lung cancer. Nat Rev Cancer. 2007 Mar;7(3): 169-81).

Treatment resistance arises frequently, often due to the secondary T790M mutation within the ATP site of the receptor. Some developed mutant-selective irreversible inhibitors are highly active against the T790M mutant, but their efficacy can be compromised by acquired mutation of C797S, that is the cysteine residue with which they form a key covalent bond (Thress, K. S. et al. Acquired EGFR C797S mutation mediates resistance to AZD9291 in non-small cell lung cancer harboring EGFR T790M. Nat. Med. 21, 560-562 (2015)). C797S mutation was further reported by Wang to be a major mechanism for resistance to T790M-targeting EGFR inhibitors (Wang et al. EGFR C797S mutation mediates resistance to third-generation inhibitors in T790M-positive non-small cell lung cancer, J Hematol Oncol. 2016; 9: 59). Additional mutations that cause resistance to Osimertinib are described by Yang, for example L718Q (Yang et al, Investigating Novel Resistance Mechanisms to Third-Generation EGFR Tyrosine Kinase Inhibitor Osimertinib in Non-Small Cell Lung Cancer Patients, Clinical Cancer Research, DOI: 10.1158/1078-0432.CCR-17-2310). Lu et al.( Targeting EGFRL858R/T790M and EGFRL858R/T790M/C797S resistance mutations in NSCLC: Current developments in medicinal chemistry, Med Res Rev 2018; 1-32) report in a review article on Targeting EGFR^{L158R/T790M} and EGFR^{L858R/T790M/C797S} resistance mutations in NSCLC treatment.

As most available EGFR tyrosine kinase inhibitors target the ATP-site of the kinase, there is a need for new therapeutic agents that work differently, for example through targeting drug-resistant EGFR mutants.

Recent studies suggest that purposefully targeting allosteric sites might lead to mutant-selective inhibitors (Jia et al. Overcoming EGFR(T790M) and EGFR(C797S) resistance with mutant-selective allosteric inhibitors, June 2016, Nature 534, 129-132).

There is just a need in the generation of selective molecules that specifically inhibit T790M/L858R, T790M/L858R/C797S, L858R, L858R/C797S containing EGFR mutants useful for the therapeutic and/or prophylactic treatment of cancer, in particular T790M and C797S containing EGFR mutants. Such inhibitors have been disclosed, for example, in WO 2018/220149.

The compound of formula (I) as described herein does have improved EGFR potency and selectivity for T790M/L858R, T790M/L858R/C797S, L858R, L858R/C797S containing EGFR mutants, in particular T790M and C797S containing EGFR mutants as well as improved physicochemical properties.

The term "pharmaceutically acceptable salt" refers to those salts of the compound of formula (I) which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition, these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compound of formula (I) are the hydrochloride salts, methanesulfonic acid salts and citric acid salts.

The abbreviation uM means microMolar and is equivalent to the symbol µM.

The abbreviation uL means microliter and is equivalent to the symbol µL.

The abbreviation ug means microgram and is equivalent to the symbol µg.

The compound of formula (I) can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Also an embodiment of the present invention is a compound according to formula (I) as described herein and pharmaceutically acceptable salts thereof, in particular a compound according to formula (I) as described herein.

Also an embodiment of the present invention is a compound according to formula (I) as described herein.

Also an embodiment of the present invention is a compound according to formula (I) as described herein, wherein the compound is 2-[4-chloro-6-[2-[4-[[4-(hydroxymethyl)-1-piperidyl]methyl]phenyl]ethynyl]-1-oxo-isoindolin-2-yl]-2-(6,7-dihydro-5H-pyrrolo[1,2-c]imidazol-1 -yl)-N-thiazol-2-yl-acetamide.

The invention thus also relates to a process for the preparation of a compound according to the invention, comprising the coupling of a compound of formula (B 1) 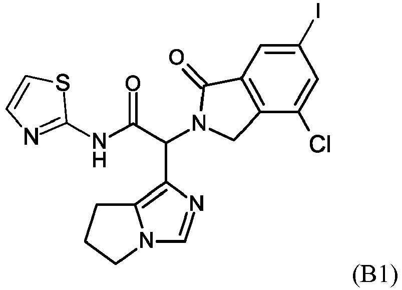 with a compound of formula (B2) 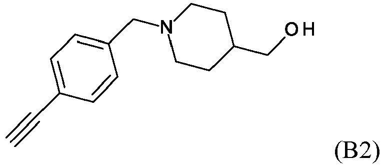 in the presence of a base and a catalyst.

The coupling can conveniently be carried out in a solvent. The solvent can be for example DMF.

In the coupling the base can be for example trimethylamine, triethylamine, dimethylamine, diethylamine or diisopropylethylamine. Conveniently the base is triethylamine.

In the coupling the catalyst is Pd(II) with suitable ligands and Cu(I). A convenient ligand is TPP.

Convenient conditions for the coupling can be between around 20°C to around 120°C, particularly between around 40°C to around 100°C, more particularly between around 60°C to around 90°C.

Preferred conditions for the coupling are the use of triethylamine in DMF at around 80°C for between around 1 h to around 24 hrs, in particular between around 2 hrs to around 5 hrs.

Processes for the manufacture of a compound of formula (I) as described herein are also an object of the invention.

The preparation of compound of formula (I) of the present invention may be carried out in sequential or convergent synthetic routes. Syntheses of the invention are shown in the following general scheme. The skills required for carrying out the reactions and purifications of the resulting products are known to those skilled in the art.

In more detail, the compound of formula (I) can be manufactured by the methods given below, by the methods given in the examples or by analogous methods. Appropriate reaction conditions for the individual reaction steps are known to a person skilled in the art. The reaction sequence is not limited to the one displayed in scheme 1, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

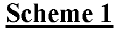

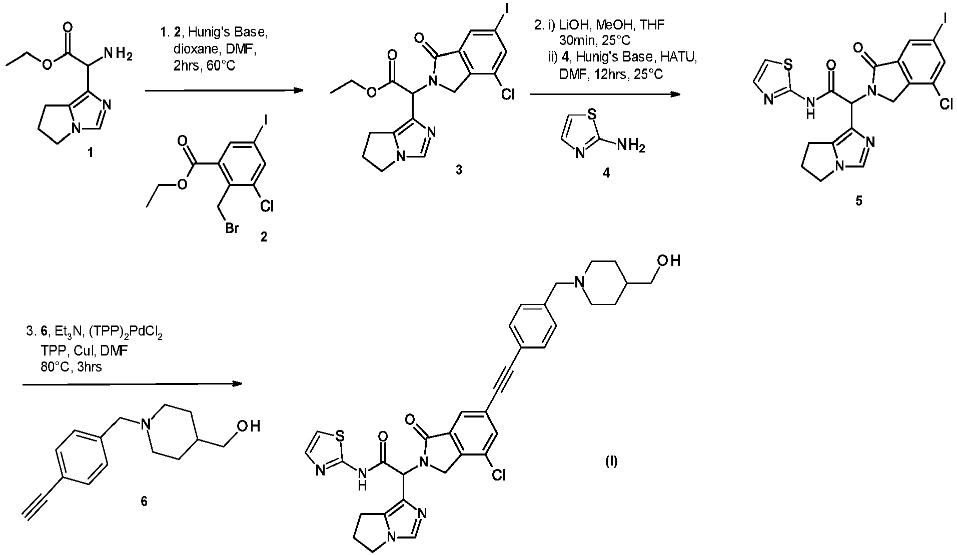

The compound of formula (I) can be obtained for example by ring cyclization of a previously prepared aminoester **1** with an appropriately substituted methyl 2-(bromomethyl)benzoate of formula **2** to yield the desired isoindoline ester **3**. Saponification and amide coupling with an appropriately substituted amine of formula **4** with a coupling agent such as HATU yields the desired amide compound of formula **5**. Sonogashira coupling of **5** with an appropriately substituted acetylene of formula **6** yields the desired isoindoline compound of formula (I) (scheme 1).

Generally speaking, the sequence of steps used to synthesize the compound of formula (I) and further functionalization can also be modified in certain cases.

Insofar as its preparation is not described in the examples, the compound of formula (I) as well as all intermediate products can be prepared according to analogous methods or according to the methods set forth herein. Starting materials are commercially available, known in the art or can be prepared by methods known in the art or in analogy thereto.

It will be appreciated that the compound of general formula (I) in this invention may be derivatised at functional groups to provide derivatives which are capable of conversion back to the parent compound *in vivo.*

A certain embodiment of the invention relates to the compound of formula (I) as described herein, or a pharmaceutically acceptable salt thereof, for use as therapeutically active substance.

A certain embodiment of the invention relates to the compound of formula (I) as described herein, or a pharmaceutically acceptable salt thereof, for the use in the therapeutic and/or prophylactic treatment of cancer, in particular non-small-cell lung cancer.

A certain embodiment of the invention relates to the compound of formula (I) as described herein, or a pharmaceutically acceptable salt thereof, for the use in the therapeutic and/or prophylactic treatment of non-small-cell lung cancer.

A certain embodiment of the invention relates to the compound of formula (I) as described herein, or a pharmaceutically acceptable salt thereof, for the manufacture of a medicament for the therapeutic and/or prophylactic treatment of cancer, in particular non-small-cell lung cancer.

A certain embodiment of the invention relates to a pharmaceutical composition comprising the compound of formula (I) as described herein, or a pharmaceutically acceptable salt thereof, and a pharmaceutically acceptable excipient.

Disclosed but not claimed is a method for the therapeutic and/or prophylactic treatment of cancer, in particular non-small-cell lung cancer by administering the compound of formula (I) as described herein, or a pharmaceutically acceptable salt thereof, to a patient.

The invention also relates in particular to:
A compound of formula (I) for use as therapeutically active substance;
A pharmaceutical composition comprising a compound of formula (I) and a therapeutically inert carrier;
A compound of formula (I) for use in the treatment or prophylaxis of cancer;
A compound of formula (I) for use in the treatment or prophylaxis of non-small cell lung cancer;
Disclosed but not claimed is the use of a compound of formula (I) for the treatment or prophylaxis of cancer;
Disclosed but not claimed is the use of a compound of formula (I) for the treatment or prophylaxis of cancer for the treatment or prophylaxis of non-small cell lung cancer;
The use of a compound of formula (I) for the preparation of a medicament for the treatment or prophylaxis of cancer;
The use of a compound of formula (I) for the preparation of a medicament for the treatment or prophylaxis of non-small cell lung cancer;
Disclosed but not claimed is a method for the treatment or prophylaxis of cancer, which method comprises administering an effective amount of a compound of formula (I) to a patient in need thereof; and
Disclosed but not claimed is a method for the treatment or prophylaxis of non-small cell lung cancer, which method comprises administering an effective amount of a compound of formula (I) to a patient in need thereof.

A certain embodiment of the invention relates to the compound of formula (I) as described herein, or a pharmaceutically acceptable salt thereof, for the use as a medicament in therapeutic and/or prophylactic treatment of a patient with EGFR activating mutations suffering from cancer, in particular non-small-cell lung cancer, comprising determining the EGFR activating mutations status in said patient and then administering the compound of formula (I) as described herein, or a pharmaceutically acceptable salt thereof, to said patient.

A certain embodiment of the invention relates to the compound of formula (I) as described herein, or a pharmaceutically acceptable salt thereof, for the use as a medicament in therapeutic and/or prophylactic treatment of a patient with EGFR mutations T790M/L858R, T790M/L858R/C797S, L858R and/or L858R/C797S suffering from cancer, in particular non-small-cell lung cancer, comprising determining the EGFR activating mutations status in said patient and then administering the compound of formula (I) as described herein, or a pharmaceutically acceptable salt thereof, to said patient.

A certain embodiment of the invention relates to the compound of formula (I) as described herein, or a pharmaceutically acceptable salt thereof, for the use as a medicament in therapeutic and/or prophylactic treatment of a patient with EGFR activating mutations as determined with a cobas^{®} EGFR Mutation Test v2 suffering from cancer, in particular non-small-cell lung cancer, comprising determining the EGFR activating mutations status in said patient and then administering the compound of formula (I) as described herein, or a pharmaceutically acceptable salt thereof, to said patient.

Furthermore, the invention includes all substituents in its corresponding deuterated form, wherever applicable, of the compound of formula (I).

Furthermore, the invention includes all optical isomers, i.e. diastereoisomers, diastereomeric mixtures, racemic mixtures, all their corresponding enantiomers and/or tautomers as well as their solvates, wherever applicable, of the compound of formula (I).

The compound of formula (I) may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compound are included within this invention. The present invention is meant to encompass all such isomeric forms of these compound. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compound may be separated so that the individual enantiomers are isolated. The separation can be carried out by methods well known in the art, such as the coupling of a racemic mixture of compound to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer(s) of the compound. Chirally pure or chirally enriched compound may be prepared by chirally selective synthesis or by separation of enantiomers. The separation of enantiomers may be carried out on the final product or alternatively on a suitable intermediate.

Also an embodiment of the present invention are compound of formula (I) as described herein, when manufactured according to any one of the described processes.

### Assay procedures

The compound of formula (I) and pharmaceutically acceptable salts thereof possess valuable pharmacological properties. The compound was investigated in accordance with the test given hereinafter.

### HTRF Phospho EGFR TMLRCS assay (cellular)

### Cell line and media

BaF3-TMLRCS cell line were obtained from Crownbio (San Diego, CA, USA). Cells were maintained at 37°C, 5% CO₂ in RPMI ATCC (Gibco 31870) + 2mM Glutamine + 0.5µg/ml Puromycin supplemented with 10% fetal bovine serum (FBS) (Gibco).

### Protocol

Cells are transferred as above to Greiner Bio-One, Nr. 784-08 micro-titerplate at 20000 cells/well in 12.5 µl of growth medium/well after the plates had been pre-filled with 12.5 nl of DMSO solutions of the to be tested compound (in dose response) or DMSO only. After spinning the plates at 300 x g for 30 seconds the cells were incubated for 4 hours at 37C, 5% CO2, 95% humidity. The cells were lysed by adding to the compound mix 4 µl/well of the supplemented lysis buffer (Cis-bio, Phospho-EGFR HTRF kit, 64EG1PEH), followed by incubation for 30 min at room temperature with shaking (400 rpm). The plates were then frozen and stored overnight at - 80C. On the next day and after thawing the plates, 4 µl of a mixture of anti-Phospho-EGFR Cryptate and of anti-Phospho-EGFR-d2 antibody solutions prepared in the supplied detection buffer was added to each well. The lidded plates were then incubated for 4 h at room temperature before reading the fluorescence emission at 616 and 665 nm using an Envision reader (Perkin Elmer). Data was analyzed in similar fashion as above using the normalized ratio of the 665 to 616 signals multiplied by 10000. The results are shown in Table 1. (A hydrogen-atom attached to the amide nitrogen is not shown).

**Table 1: BaF3 cellular HTRF Phospho EGFR TMLRCS assay data**

| **Exam.** | **Structure** | **IC₅₀** (BaF3 TMLRCS) |
|---|---|---|
| 1 | 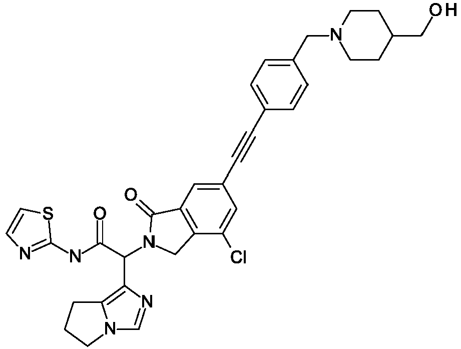 2-[4-chloro-6-[2-[4-[[4-(hydroxymethyl)-1-piperidyl]methyl]phenyl]ethynyl]-1-oxo-isoindolin-2-yl]-2-(6,7-dihydro-5H-pyrrolo[1,2-c]imidazol-1-yl)-N-thiazol-2-yl-acetamide | 3nM |

The compound of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays), rectally (e.g. in the form of suppositories) or topical ocularly (e.g. in the form of solutions, ointments, gels or water soluble polymeric inserts). However, the administration can also be effected parenterally, such as intramuscularly, intravenously, or intraocularly (e.g. in the form of sterile injection solutions).

The compound of formula (I) and their pharmaceutically acceptable salts can be processed with pharmaceutically inert, inorganic or organic adjuvants for the production of tablets, coated tablets, dragées, hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

### Pharmaceutical Compositions

The compound of formula (I) or a pharmaceutically acceptable salt thereof can be used as therapeutically active substances, e.g. in the form of a pharmaceutical preparation. The pharmaceutical preparation can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compound of formula (I) or a pharmaceutically acceptable salt thereof can be processed with pharmaceutically inert, inorganic or organic carriers for the production of a pharmaceutical preparation. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparation can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula (I) or a pharmaceutically acceptable salt thereof and a therapeutically inert carrier are also provided by the present invention, as is a process for their production, which comprises bringing one or more compound of formula (I) and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration the dosage for adults can vary from about 0.01 mg to about 1000 mg per day of a compound of general formula (I) or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparations conveniently contain about 1-500 mg, particularly 1-100 mg, of a compound of formula (I). Examples of compositions according to the invention are:

### Example A

Tablets of the following composition are manufactured in the usual manner:

**Table 2: possible tablet composition**

| ingredient | mg/tablet | | | |
|---|---|---|---|---|
| | 5 | 25 | 100 | 500 |
| Compound of formula (I) | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Example B-1

Capsules of the following composition are manufactured:

**Table 3: possible capsule ingredient composition**

| ingredient | mg/capsule | | | |
|---|---|---|---|---|
| | 5 | 25 | 100 | 500 |
| Compound of formula (I) | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula (I), lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 4: possible soft gelatin capsule ingredient composition**

| ingredient | mg/capsule |
|---|---|
| Compound of formula (I) | 5 |
| Yellow wax | 8 |
| Hydrogenated Soya bean oil | 8 |
| Partially hydrogenated plant oils | 34 |
| Soya bean oil | 110 |
| Total | 165 |

**Table 5: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula (I) is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

| ingredient | mg/supp. |
|---|---|
| Compound of formula (I) | 15 |

**Table 6: possible suppository composition**

| | |
|---|---|
| Suppository mass | 1285 |
| Total | 1300 |

### Manufacturing Procedure

The suppository mass is melted in a glass or steel vessel, mixed thoroughly and cooled to 45°C. Thereupon, the finely powdered compound of formula (I) is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 7: possible injection solution composition**

| ingredient | mg/injection solution. |
|---|---|
| Compound of formula (I) | 3 |
| Polyethylene Glycol 400 | 150 |
| acetic acid | q.s. ad pH 5.0 |
| water for injection solutions | ad 1.0 ml |

### Manufacturing Procedure

The compound of formula (I) is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 8: possible sachet composition**

| ingredient | mg/sachet |
|---|---|
| Compound of formula (I) | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula (I) is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Examples

The following examples are provided for illustration of the invention. They should not be considered as limiting the scope of the invention, but merely as being representative thereof.

### Abbreviations

AcOH = acetic acid; DCM = dichloromethane; DIPEA = diisopropylethylamine; DMAP = dimethylaminopyridine; DMF = dimethylformamide; DMSO = diemethyl sulfoxide; ESI = electrospray ionization; EtOAc = ethyl acetate; EtOH = ethanol; GTP = guanosine triphosphate; HATU = hexafluorophosphate azabenzotriazole tetramethyl uronium; HPLC = high performance liquid chromatography; MeOH = methanol; MS = mass spectrometry; NMP = N-methyl-2-pyrrolidone; NMR = nuclear magnetic resonance; RT = room temperature; THF = tetrahydrofuran; TPP = triphenylphosphine; TRIS = tris(hydroxymethyl)aminomethane.

### Example 1

### (2RS)-2-[4-Chloro-6-[2-[4-[[4-(hydroxymethyl)-1-piperidyl]methyl]phenyl]ethynyl]-1-oxo-isoindolin-2-yl]-2-(6,7-dihydro-5H-pyrrolo[1,2-c]imidazol-1-yl)-N-thiazol-2-yl-acetamide

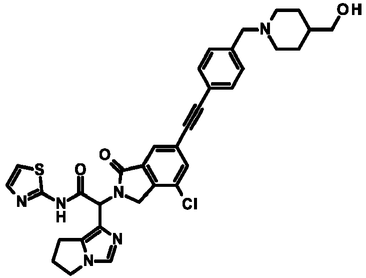

### Step 1: Ethyl 2-(6,7-dihydro-5H-pyrrolo[1,2-climidazol-1-yl)-2-oxo-acetate

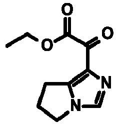

To a solution of ethyl 2-(6,7-dihydro-SH-pyrrolo[1,2-c]imidazol-1-yl)acetate (20.0 g, 102.97 mmol) dissolved in 200 ml of 1,4-dioxane was added selenium dioxide (22.85 g, 205.94 mmol, 2 equiv.). The reaction mixture was stirred for 5 hours at 80°C. The reaction mixture was concentrated under vacuum to give a residue. The crude product was purified by flash chromatography on a silica gel column eluting with petroleum ether:ethyl acetate 2:1 to ethyl acetate:ethanol 10:1 gradient to obtain the desired ethyl 2-(6,7-dihydro-5H-pyrrolo[1,2-c]imidazol-1-yl)-2-oxo-acetate (quant. yield) as a light brown oil, MS: m/e = 209.1 (M+H⁺).

### Step 2: Ethyl 2-(6,7-dihydro-5H-pyrrolo[1,2-c]imidazol-1-yl)-2-hydroxyimino-acetate

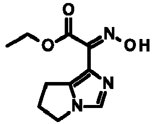

To a solution of ethyl 2-(6,7-dihydro-5H-pyrrolo[1,2-c]imidazol-1-yl)-2-oxo-acetate *(Example 1, step 1)* (17.5 g, 84.05 mmol) dissolved in 145 ml of ethanol was added hydroxylamine hydrochloride (6.42 g, 92.45 mmol, 1.1 equiv.) and sodium acetate (13.79 g, 168.1 mmol, 2 equiv.) at room temperature. The reaction mixture was stirred for 3.5 hours at 80°C. The reaction mixture was concentrated and extracted with water and five times with a mixture of ethanol/THF/ethyl acetate 1:1:8. The organic layers were concentrated to dryness. The desired ethyl 2-(6,7-dihydro-5H-pyrrolo[1,2-c]imidazol-1-yl)-2-hydroxyimino-acetate (15 g, 80 % yield) was obtained as a yellow solid, MS: m/e = 224.1 (M+H⁺) and used directly in the next step.

### Step 3: Ethyl (2RS)-2-amino-2-(6,7-dihydro-5H-pyrrolo[1,2-c]imidazol-1-yl)acetate

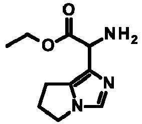

To a solution of ethyl 2-(6,7-dihydro-5H-pyrrolo[1,2-c]imidazol-1-yl)-2-hydroxyimino-acetate *(Example 1, step 2)* (15.0 g, 67.2 mmol) dissolved in 225 ml of ethanol and 120 ml of THF was added Pd/C (30.0g, 67.2 mmol, 1 eq, 10%) at room temperature. The mixture was hydogenated with H₂ for 24 hours at 45°C. The reaction mixture was filtered and the filtrate was concentrated under vacuum. The desired ethyl (2*RS*)-2-amino-2-(6,7-dihydro-5H-pyrrolo[1,2-c]imidazol-1-yl)acetate (quant, yield) was obtained as a brown oil, MS: m/e = 210.1 (M+H⁺) and used directly in the next step.

### Step 4: Ethyl (2RS)-2-amino-2-(6,7-dihydro-5H-pyrrolo[1,2-c]imidazol-1-yl)acetate hydrochloride

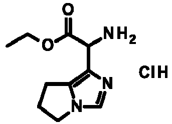

A solution of ethyl (2*RS*)-2-amino-2-(6,7-dihydro-5H-pyrrolo[1,2-c]imidazol-1-yl)acetate *(Example 1, step 3)* (15.0 g, 82.79 mmol) in HCl/EtOH (300 ml, 1200 mmol, 14.5 equiv., 2.5 mol/L) was stirred at 25 °C for 36 hours. The reaction mixture was concentrated under vacuum below 25°C to give a residue as brown oil. 150 ml of acetonitrile were added to the residue and the precipitated yellow solid was collected and dried under vacuum below 25 °C to give the desired ethyl (2*RS*)-2-amino-2-(6,7-dihydro-5H-pyrrolo[1,2-c]imidazol-1-yl)acetate hydrochloride (quant. yield) as yellow solid, MS: m/e = 210.1 (M+H⁺).

### Step 5: Ethyl 2-(bromomethyl)-3-chloro-5-iodo-benzoate

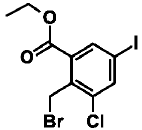

3-Chloro-5-iodo-2-ethyl-benzoate (57.3 g, 176 mmol) was dissolved in 400 ml tetrachloroethylene and N-bromosuccinimide (46.9 g, 265 mmol, 1.5 equiv.) and AIBN (13.4 g, 88.3 mmol, 0.5 equiv.) were added at room temperature. The mixture was stirred at 80°C for 16 hours. The reaction mixture was concentrated to dryness. The crude product was purified by flash chromatography on a silica gel column eluting with a petroleum ether:ethyl acetate 1:0 to 10:1 gradient to obtain the desired product (56 g, 76 % yield) as a pink solid.

### Step 6: Ethyl (2RS)-2-(4-chloro-6-iodo-1-oxo-isoindolin-2-yl)-2-(6,7-dihydro-5H-pyrrolo[1,2-c]imidazol-1-yl)acetate

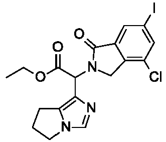

Ethyl (2*RS*)-2-amino-2-(6,7-dihydro-5H-pyrrolo[1,2-c]imidazol-1-yl)acetate hydrochloride *(Example 1, step 4)* (12 g, 48.8 mmol, 1 equiv.) was dissolved in 120 ml of dioxane and 20 ml of DMF. Ethyl 2-(bromomethyl)-3-chloro-5-iodo-benzoate *(Example 1, step 5)* (19.7 g, 48.8 mmol) and diisopropylethylamine (34 ml, 195 mmol, 4 equiv.) were added at room temperature. The mixture was stirred at room temperature for 30 minutes and at 60°C for 2 hours. The reaction mixture was extracted with water and two times with ethyl acetate. The organic layers were extracted with brine, dried over sodium sulfate and concentrated to dryness. The crude product was purified by flash chromatography on a silica gel column eluting with an ethyl acetate:methanol 100:0 to 90: 10 gradient to obtain the desired product (14.5 g, 55 % yield) as a light red solid, MS: m/e = 486.2 (M+H⁺).

### Step 7: (2RS)-2-(4-Chloro-6-iodo-1-oxo-isoindolin-2-yl)-2-(6,7-dihydro-5H-pyrrolo[1,2-c]imidazol-1-yl)-N-thiazol-2-yl-acetamide

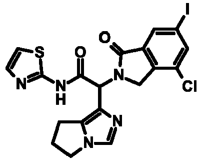

Ethyl (2RS)-2-(4-chloro-6-iodo-1-oxo-isoindolin-2-yl)-2-(6,7-dihydro-5H-pyrrolo[1,2-c]imidazol-1-yl)acetate *(Example 1, step 6)* (14.5 g, 29.9 mmol) was dissolved in 70 ml of methanol and 70 ml of THF. LiOH (1M in water) (32.8 ml, 32.8 mmol, 1.1 equiv.) was added at room temperature. The mixture was stirred for 30 minutes at room temperature. The reaction mixture was concentrated in vacuo to dryness and the residue was dissolved in 140 ml of DMF. Thiazol-2-amine (3.1 g, 31.3 mmol, 1.05 equiv.), Hunig's base (15.6 ml, 89.6 mmol, 3 equiv.) and HATU (13.6 g, 35.8 mmol, 1.2 equiv.) were added at room temperature. The mixture was stirred at room temperature for 90 minutes. The reaction mixture was extracted with water and two times with ethyl acetate. The organic layers were extracted with water, dried over sodium sulfate and concentrated to dryness. The crude product was purified by flash chromatography on a silica gel column eluting with a dichloromethane:methanol 100:0 to 90:10 gradient to obtain the desired product (10.5 g, 59 % yield) as a light brown solid, MS: m/e = 540.1 (M+H⁺).

### Step 8: [1-1(4-Ethynylphenyl)methyl]-4-piperidyl]methanol

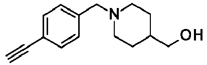

4-Ethynylbenzaldehyde (500 mg, 3.84 mmol) was dissolved in 3 ml of dichloromethane. Piperidin-4-ylmethanol (490 mg, 4.23 mmol, 1.1 equiv.) and sodium triacetoxyborohydride (1.3 g, 6.15 mmol, 1.6 equiv.) were added at room temperature. The mixture was stirred at room temperature for 1.5 hours. The reaction mixture was extracted with water and two times with dichloromethane. The organic layers were extracted with brine, dried over sodium sulfate and concentrated to dryness. The crude product was purified by flash chromatography on a silica gel column eluting with a dichloromethane:methanol 100:0 to 90:10 gradient to obtain the desired product (900 mg, 94 % yield) as an orange oil, MS: m/e = 230.2 (M+H⁺).

### Step 9: (2RS)-2-[4-Chloro-6-[2-[4-[[4-(hydroxymethyl)-1-piperidyl]methyl]phenyl]ethynyl]-1-oxo-isoindolin-2-yl]-2-(6,7-dihydro-5H-pyrrolo[1,2-c]imidazol-1-yl)-N-thiazol-2-yl-acetamide

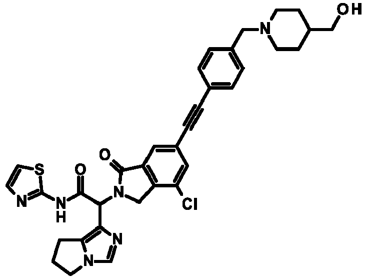

(2RS)-2-(4-Chloro-6-iodo-1-oxo-isoindolin-2-yl)-2-(6,7-dihydro-5H-pyrrolo[1,2-c]imidazol-1-yl)-N-thiazol-2-yl-acetamide *(Example 1, step 7)* (70 mg, 0.13 mmol) and [1-[(4-ethynylphenyl)methyl]-4-piperidyl]methanol *(Example 1, step 8)* (39 mg, 0.17 mmol, 1.3 equiv.) were dissolved in 1 ml of DMF. Triethylamine (0.05 ml, 0.39 mmol, 3 equiv.), bis-(triphenylphosphine)-palladium(II)dichloride (4.6 mg, 0.007 mmol, 0.05 equiv.), triphenylphosphine (3.4 mg, 0.013 mmol, 0.1 equiv.) and copper(I)iodide (1 mg, 0.007 mmol, 0.05 equiv.) were added and the mixture was stirred for 3 hours at 80°C. The reaction mixture was extracted with water and two times with ethyl acetate. The organic layers were extracted with brine, dried over sodium sulfate and concentrated to dryness. The crude product was purified by flash chromatography on a silica gel column eluting with a dichloromethane:methanol 100:0 to 90:10 gradient to obtain the desired product (26 mg, 30 % yield) as a white solid, MS: m/e = 641.4 (M+H⁺).

## Claims

1. A compound of formula (I) 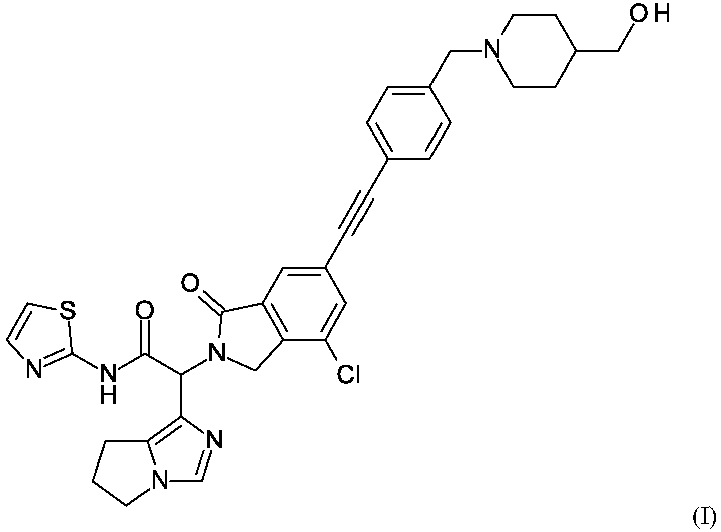 or a pharmaceutically acceptable salt thereof.

2. A compound according to claim 1, wherein the compound is the compound of formula (I).

3. A compound according to claim 1 or 2, wherein the compound is 2-[4-chloro-6-[2-[4-[[4-(hydroxymethyl)-1-piperidyl]methyl]phenyl]ethynyl]-1-oxo-isoindolin-2-yl]-2-(6,7-dihydro-5H-pyrrolo[1,2-c]imidazol-1-yl)-N-thiazol-2-yl-acetamide.

4. A process for the preparation of a compound according to any one of claims 1 to 3, comprising the coupling of a compound of formula (B1) 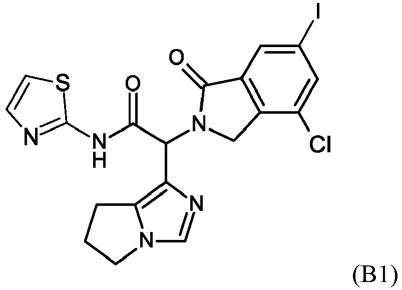 with a compound of formula (B2) 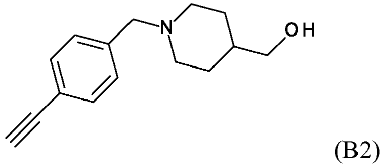 in the presence of a base and a catalyst.

5. A compound according to claim 1 or 3 for use as therapeutically active substance.

6. A pharmaceutical composition comprising a compound according to claim 1 or 3 and a therapeutically inert carrier.

7. A compound according to claim 1 or 3 for use in the treatment or prophylaxis of cancer.

8. A compound for use according to claim 7 wherein the cancer is non-small cell lung cancer.

## Patentansprüche

1. Verbindung der Formel (I) 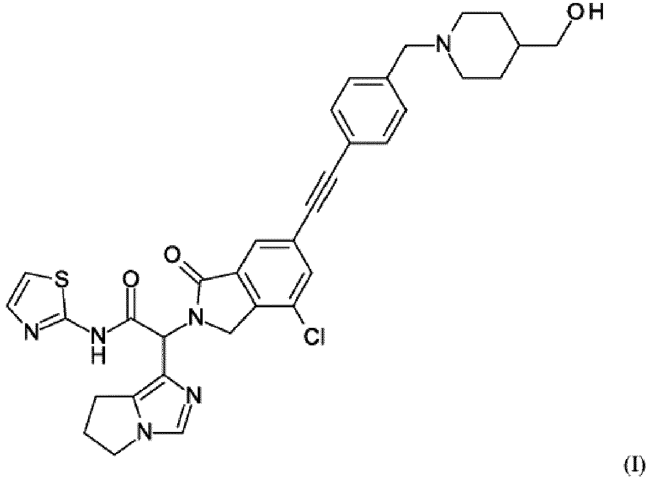 oder ein pharmazeutisch unbedenkliches Salz davon.

2. Verbindung nach Anspruch 1, wobei die Verbindung die Verbindung der Formel (I) ist.

3. Verbindung nach Anspruch 1 oder 2, wobei die Verbindung 2-[4-Chlor-6-[2-[4-[[4-(hydroxymethyl)-1-piperidyl]methyl]phenyl]ethinyl]-1-oxo-isoindolin-2-yl]-2-(6,7-dihydro-5H-pyrrolo[1,2-c]imidazol-1-yl)-N-thiazol-2-yl-acetamid ist.

4. Verfahren zur Herstellung einer Verbindung nach einem der Ansprüche 1 bis 3, umfassend das Koppeln einer Verbindung der Formel (B1) 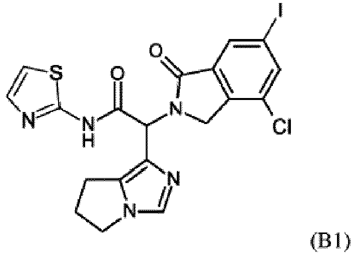 mit einer Verbindung der Formel (B2) 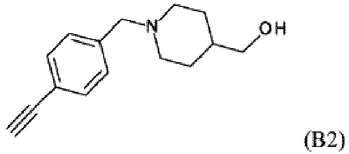 in der Gegenwart einer Base und eines Katalysators.

5. Verbindung nach Anspruch 1 oder 3 zur Verwendung als therapeutisch wirksame Substanz.

6. Pharmazeutische Zusammensetzung, umfassend eine Verbindung nach Anspruch 1 oder 3 und einen therapeutisch inerten Träger.

7. Verbindung nach Anspruch 1 oder 3 zur Verwendung bei der Behandlung oder Prophylaxe von Krebs.

8. Verbindung zur Verwendung nach Anspruch 7, wobei der Krebs nicht-kleinzelliger Lungenkrebs ist.

## Revendications

1. Composé de formule (I) 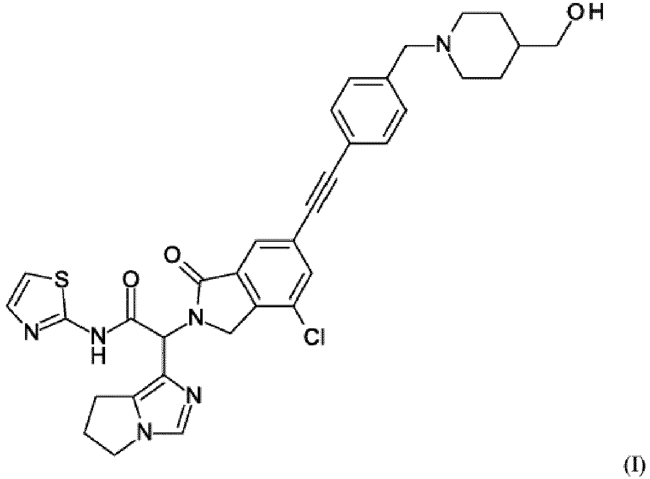 ou un sel pharmaceutiquement acceptable de celui-ci.

2. Composé selon la revendication 1, dans lequel le composé est le composé de formule (I).

3. Composé selon la revendication 1 ou 2, dans lequel le composé est le 2-[4-chloro-6-[2-[4-[[4-(hydroxyméthyl)-1-pipéridyl]méthyl]phényl]éthynyl]-1-oxo-isoindolin-2-yl]-2-(6,7-dihydro-5H-pyrrolo[1,2-c]imidazol-1-yl)-N-thiazol-2-yl-acétamide.

4. Procédé de préparation d'un composé selon l'une quelconque des revendications 1 à 3, comprenant le couplage d'un composé de formule (B1) 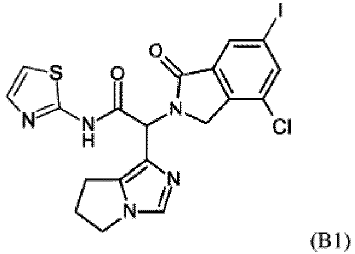 avec un composé de formule (B2) 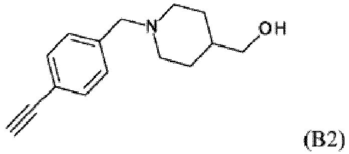 en présence d'une base et d'un catalyseur.

5. Composé selon la revendication 1 ou 3 pour une utilisation comme substance thérapeutiquement active.

6. Composition pharmaceutique comprenant un composé selon la revendication 1 ou 3 et un véhicule thérapeutiquement inerte.

7. Composé selon la revendication 1 ou 3 pour une utilisation dans le traitement ou la prophylaxie d'un cancer.

8. Composé pour une utilisation selon la revendication 7 dans lequel le cancer est un cancer du poumon non à petites cellules.
